# EUROPÄISCHE PATENTSCHRIFT

(11) **EP 1 379 198 B1**
(45) Veröffentlichungstag und Bekanntmachung des Hinweises auf die Patenterteilung: **27.07.2011**
(21) Anmeldenummer: 02732374.0
(22) Anmeldetag: 05.04.2002
(51) Int. Cl.: A61F 2/06

(54) **ENTFERNBARE, IM WESENTLICHEN ZYLINDRISCHE IMPLANTATE**
REMOVABLE, ESSENTIALLY CYLINDRICAL IMPLANTS
IMPLANTS AMOVIBLES, SENSIBLEMENT CYLINDRIQUES

(30) Priorität: 18.04.2001 DE 10118944
(43) Veröffentlichungstag der Anmeldung: 14.01.2004
(73) Patentinhaber: Merit Medical Systems, Inc., South Jordan UT 84095 (US)
(72) Erfinder: FREITAG, Lutz, 58675 Hemer (DE)
(74) Vertreter: Griepenstroh, Jörg
(86) Internationale Anmeldenummer: PCT/DE2002/001244
(87) Internationale Veröffentlichungsnummer: WO 2002/083037

(56) Entgegenhaltungen:
- WO-A-00/44308
- WO-A-94/22379
- DE-A- 19 703 482
- US-A- 5 035 706
- US-A- 5 035 706
- US-A- 5 776 186
- US-A- 5 776 186

## Beschreibung

Die Erfindung betrifft entfernbare, im wesentlichen zylindrische Implantate.

Implantate zum Einsetzen in den Körper werden beispielsweise in der WO 00/44308 A als eine Vorrichtung aus mehreren Formgedächtnisdrähten beschrieben, die miteinander verwebt sind und den Implantatkörper bilden. Der Implaritatkörper besitzt ein erstes und ein zweites Ende. Die Formgedächtnisdrähte überkreuzen einander und bilden dadurch mehrere Winkel aus, wobei wenigstens einer der Winkel stumpf ist und beide Ende von wenigstens einem Formgedächtnisdraht in der Mitte von einem Ende des Körpers angeordnet sind. Dabei ist der Wert von dem wenigstens einem stumpfen Winkel dadurch vergrößert, dass der Körper axial zusammengedrückt wird. Dadurch wird der Implantatkörper beim Verringern seines Durchmessers länger. Die US 5 776 186 A hingegen offenbart drahtförmige Gebilde, welche die Implantate umschlingen und bereits an einem Ende eine Fangvorrichtung aufweisen. Durch diese drahtförmigen Gebilde wird das Implantat expandiert. Durch einen Zug an den Fangvorrichtungen kann der Stent insgesamt verlagert werden. Die US 5 035 706 A beschreibt eine Implantatausführung, bestehend aus Drahtkonfigurationen, welche zickzackförmig angeordnet sind, wobei jeweils aneinander grenzende Enden der Zickzackstreben verbunden sind und wobei an diesen Enden Ösen vorgesehen sind, durch welche ein Faden hindurchgezogen ist. Durch Zug an diesem Faden wird der Abstand zwischen den Ösen verringert und die Zickzackstruktur quasi zusammengezogen, so dass der Durchmesser des implantats verringert wird.

Derartige Implantate, also im überwiegenden Teil sogenannte Stents, müssen aus verschiedensten Gründen nach den operativen Einsätzen gelegentlich wieder aus dem Körper entfernt werden, was in vielen Fällen nicht unproblematisch ist, da neugebildetes Gewebe an die Implantate heran und - je nach deren Struktur - auch hindurchwächst und daher die Entfernung zu Komplikationen Veranlassung geben kann.

Es besteht daher noch ein Bedürfnis danach, derartige zylindrische Implantate so auszugestalten, daß sie ohne besonderen Aufwand und möglichst komplikationslos aus dem Körper wieder entfernt werden können.

Zur Lösung der Aufgabe werden entfernbare zylindrische Implantate, insbesondere Stents entsprechend Hauptanspruch vorgeschlagen.

Überraschenderweise hat sich herausgestellt, daß die bisherigen Schwierigkeiten bei der Entfernung entsprechender Implantate in relativ einfacher Weise behoben werden können, wenn diese Implantate in ihrem Durchmesser reduzierbar sind und von einem oder mehreren dünnen elastischen drahtförmigen Gebilden, im folgenden der Einfachheit halber als Draht bezeichnet, umschlungen sind und dieser Draht an mindestens einem Ende eine Fangvorrichtung aufweist, in die mit geeignetem chirurgischen Gerät eingegriffen bzw. die von einem solchen Gerät umfaßt werden kann. Durch Zug an der Fangvorrichtung wird der Durchmesser des Implantates durch den Draht in einer oder mehreren Höhen des Gesamtimplantates verringert, so daß sich das

verringert, so daß sich das Implantat von dem Gewebe oder von den Gefäßwandungen löst und herausgezogen werden kann.

Die erfindungsgemäß einsetzbaren Implantate, also insbesondere Stents, bestehen aus gitter- oder netzförmigen physiologisch verträglichem Metall oder Kunststoff und sind häufig mindestens an einer Seite mit einer offenen Nut versehen, so daß diese im letzteren Falle zusammendrückbar, aber sonst auseinderziehbar und damit auf jeden Fall im Durchmesser reduzierbar sind. Dies wird erfindungsgemäß dadurch erreicht, daß ein oder mehrere Drähte aus Metall oder Kunststoff, die dünn und elastisch sind, an mindestens einer Höhe des Implantates um dieses herumgeschlungen und in der Regel wegen der erhöhten Sicherheit an einem Ende des Implantates beispielsweise durch übliche Befestigungstechniken wie Schweißen, Umschlingen, Verknoten oder in ähnlicher Weise befestigt werden. Es ist aber auch möglich, in bestimmten Fällen auf eine solche Befestigung zu verzichten, wenn der Draht das Implantat auf einer Höhe mehrfach umschließt und daher nicht abgleiten kann. Mindestens ein Ende des Drahtes oder der Drähte ist mit einer Fangvorrichtung versehen, die beispielsweise als verdicktes Ende zum Umfassen mit einem chirurgischen Gerät oder als Öse oder Schlinge zum Eingreifen mit geeignetem chirurgischen Gerät ausgebildet sein kann.

In einer bevorzugten Ausführungsform wird das Implantat aber mindestens von 2 Drähten umschlungen, die spiralförmig um das Implantat gewickelt sind und bei denen beide freien, daß heißt also nicht befestigten Enden, über eine entsprechende Fangvorrichtung verfügen.

Wesentlich ist es auf jeden Fall, daß die Fangvorrichtung bereits bei der Konstruktion des Implantates vorgesehen ist, d.h., daß später nicht eine Schlinge um ein Implantat herumgelegt werden muß, sondern daß diese bereits bei der Produktion des Implantates vorgesehen bzw. eingearbeitet wird. Der Draht verläuft frei um das Implantat, insbesondere bei mehrfacher Wicklung oder er kann in Führungsösen verlaufen. Der Draht verläuft zwischen dem Geflecht oder Gitter des Implantates und einem äußeren Mantel. Wenn die Schlinge außen angebracht ist, führt dies dazu, daß das Implantat zusammengedrückt bzw. zusammengeschnürt wird, insbesondere, wenn eine Nut im Implantat vorgesehen ist. Wird der Zugdraht innen angeordnet, zieht er den Stent in sich zusammen. Entscheidend ist auf jeden Fall, daß der axiale Durchmesser durch den longitudinalen Zug verringert wird, so daß das Implantat sich vorsichtig vom Gewebe lösen kann.

Die Drahtkonstruktion kann über die gesamte Länge des Stents oder nur am oberen Ende verlaufen. Ggf. kann die Drahtkonstruktion auch durch das Lumen des Stents verlaufen, dann beispielsweise über Kreuz.

Bei Metallimplantaten oder der Verwendung von Metalldrähten wird die Konstruktion vorzugsweise so ausgestaltet, daß sie endoskopisch unter Sicht oder unter Röntgenkontrolle ohne direkte Sicht erfaßt werden kann.

Durch die erfindungsgemäße Konstruktion wird die Entfernung von zylindrischen Implantaten stark erleichtert, da der Zeitaufwand und die Komplikationen deutlich geringer sind.

Im folgenden wird die Erfindung anhand der Zeichnungen näher erläutert:

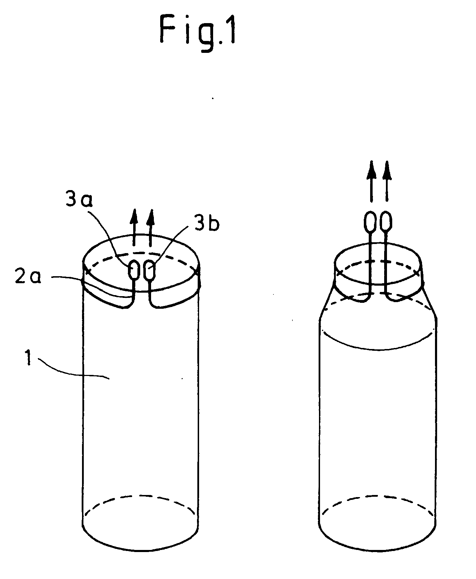

- Fig.1: zeigt in schematischer Darstellung ein Implantat mit einer einfachen Schleife an einem Ende
- Fig. 2: zeigt in schematischer Darstellung ein Implantat mit einer Spiralschleife als Einzelschleife
- Fig. 3: zeigt in schematischer Darstellung ein Implantat mit Spriralschleife als Einzelschleife und einer einfachen Schleife am Ende
- Fig. 4: zeigt ebenfalls in schematischer Darstellung ein Implantat mit Spiralschleifen als Doppelschleife.

Das Implantat 1 in im wesentlichen zylindrischer Form ist entweder an einem Ende mit einer drahtförmigen Einzelschleife umschlungen, die entweder an der Außenwand des Implantates befestigt sein kann oder die das Implantat mehrfach umschlingt. Diese drahtförmige Schleife 2a weist an jedem Ende eine Fangvorrichtung 3a bzw. 3b auf, die bei Zug in Richtung der angedeuteten Pfeile mindestens teilweise zu einer Reduzierung des Durchmessers des Implantates führt.

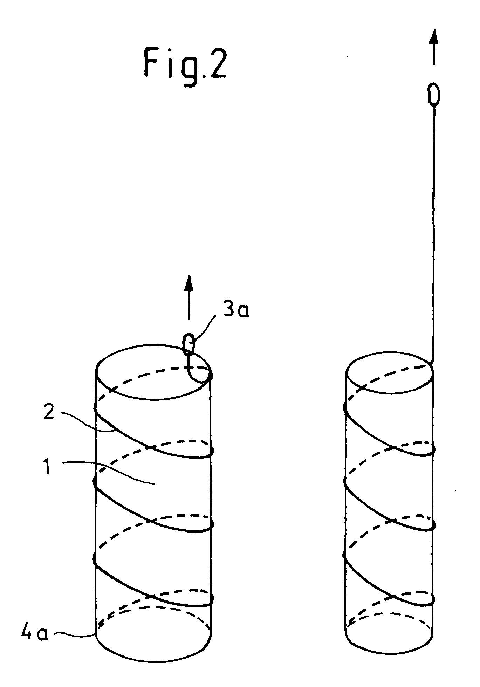

Anstelle einer einfachen Schleife kann der Draht auch, wie in Fig. 2 dargestellt, als Spiralschleife 2 ausgeführt sein, die über eine Fangvorrichtung 3a verfügt und am Implantat an deren Ende 4a befestigt ist, so daß bei Zug in der angegebenen Pfeilrichtung eine Verringerung des Gesamtdurchmessers des Implantates stattfindet.

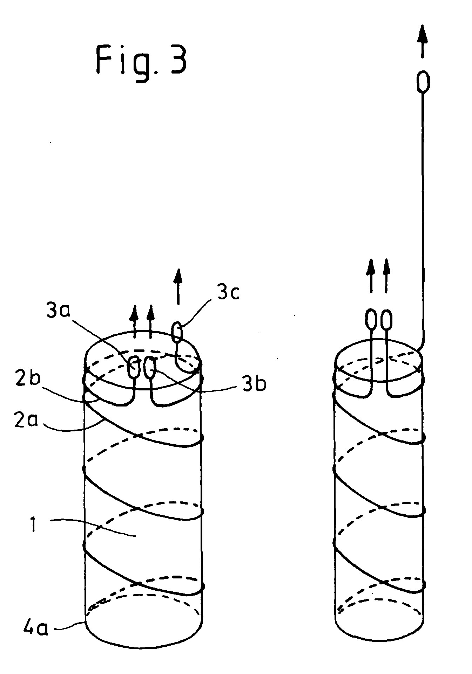

Wie in Fig. 3 gezeigt, können auch eine Spiralschleife und eine einfache Schleife miteinander kombiniert werden, indem das Implantat 1 einerseits über eine Spiralschleife 2a verfügt, die am Ende 4a befestigt ist, sowie andererseits über eine einfache Schleife 2b, wobei beide Schleifen mit Fangvorrichtungen 3a, 3b und 3c versehen sind. Bei Zug erfolgt daher eine Verringerung des Gesamtdurchmessers, die im oberen Ende der zusätzlichen Einfachschleife 2b stärker ausgeprägt ist als im übrigen Teil.

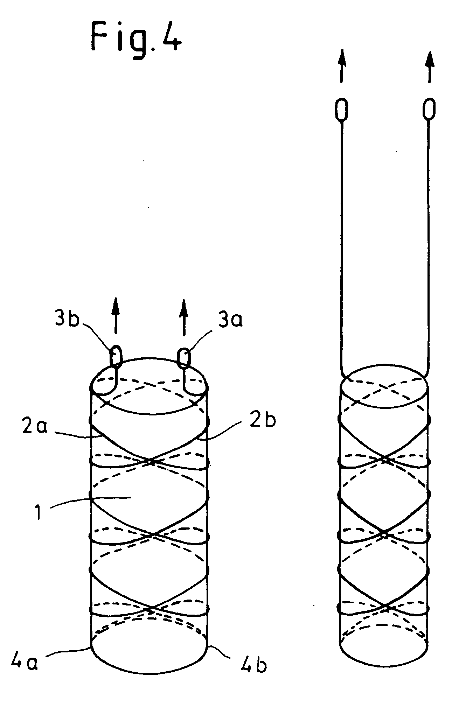

In einem Beispiel, welches in Fig. 4 dargestellt ist, weist das Implantat 1 zwei Spiralschleifen 2a und 2b auf, die an den Enden 4a und 4b des Implantates befestigt sind und in Fangvorrichtungen 3a und 3b enden. Durch die doppelte Spiralschleife läßt sich eine besonders ausgeprägte Reduktion des Durchmessers des Implantates erzielen.

## Patentansprüche

1. Entfernbare, im wesentlichen zylindrische Implantate, welche ein- oder mehrfach in einer oder mehreren Höhen von einem oder mehreren elastischen, dünnen, drahtförmigen Gebilden (2a, 2b) umschlungen sind, das oder die an einem Ende eine Fangvorrichtung (3a, 3b) aufweist bzw. aufweisen, wobei ein Ende (4a, 4b) oder mehrere Enden (4a, 4b) des drahtförmigen Gebildes (2a, 2b) oder der drahtförmigen Gebilde (2a, 2b) am oder im Implantat gefestigt ist/sind, wobei die Schlaufen frei um das Implantat verlaufen, wobei wenigstens eine Schlaufe des oder der drahtförmigen Gebilde (2a, 2b) dafür vorgesehen ist, bei Zug an den Fangvorrichtungen (3a, 3b) zumindest teilweise den Durchmesser des Implantats zu verringern und wobei das oder die drahtförmigen Gebilde (2a, 2b) zwischen dem Implantat und einem äußeren Mantel verlaufen.

2. Zylindrische Implantate nach Anspruch 1, **dadurch gekennzeichnet, dass** das Implantat (1) aus einem ggf. mit einer offenen Nut versehenen Gitter oder Netz aus Metall oder Kunststoffen besteht.

3. Zylindrische Implantate nach Anspruch 1 oder 2, **dadurch gekennzeichnet, dass** das oder die drahtförmige(n) Gebilde aus Metall oder Kunststoff bestehen.

4. Zylindrische Implantate nach Anspruch 1 bis 3, **dadurch gekennzeichnet, dass** die Fangvorrichtung (3a, 3b) als verdicktes Ende, Öse oder Schlinge ausgebildet ist.

5. Zylindrisches Implantat nach Anspruch 1 bis 4, **dadurch gekennzeichnet, dass** das oder die drahtförmigen Gebilde (2a, 2b) das Implantat (1) nur auf einer Höhe umfassen.

6. Zylindrische Implantate nach Anspruch 1 bis 5, **dadurch gekennzeichnet, dass** das oder die drahtförmigen Gebilde (2a, 2b) das Implantat (1) spiralförmig umschlingen.

7. Zylindrische Implantate nach Anspruch 1 bis 6, **dadurch gekennzeichnet, dass** das oder die drahtförmigen Gebilde (2a, 2b) über die gesamte Länge des Implantates (1) ausgeführt sind.

8. Zylindrische Implantate nach Anspruch 1 bis 6, **dadurch gekennzeichnet, dass** das oder die drahtförmigen Gebilde (2a, 2b) nur am oberen Ende des Implantates (1) verlaufen.

9. Zylindrische Implantate nach Anspruch 1 bis 8, **dadurch gekennzeichnet, dass** sie unter Röntgenkontrolle ohne direkte Sicht erfasst werden können.

## Claims

1. Removable, essentially cylindrical implants around which, singly or multiply, at one o rmore levels, one or more elastic, thin wire structure(s) (2a, 2b) is/are wound, which has or have, at one end, a catch element (3a, 3b), whereby one end (4a, 4b) or several ends (4a, 4b) of the wire structure (2a, 2b) orof the wire structures (2a, 2b) is/are tightened atorin the implant, where by the loops run freely around the implant, whereby at least one loop of the wire structure(s) (2a, 2b) is intended, when the catch elements (3a, 3b) are pulled, to at least partially reduce the diameter of the implant and whereby the wire structure(s) (2a, 2b) run(s) between the implant and an outer sheath.

2. Cylindrical implants according to claim 1, **characterised in that** the implant (1) consists of a lattice or mesh made of metal or plastics provided, if required, with an open groove.

3. Cylindrical implants according to claim 1 or 2, **characterised in that**the wirestructure(s)consist(s) of metalorplastic.

4. Cylindrical implants according to claim 1 to 3, **characterised in that**the catch element (3a, 3b) is shaped as a thickened end, eyeorsling.

5. Cylindrical implant according to claim 1 to 4, **characterised in that**the wire structure(s) (2a, 2b) comprise(s) the implant (1) at one level only.

6. Cylindrical implant saccording to claim 1 to 5, **characterised in that**the wire structure(s) (2a, 2b) is/are spirally wound around theimplant (1).

7. Cylindrical implants according to claim 1 to 6, **characterised in that**the wire structure(s) (2a, 2b) run(s) across the entire length of theimplant (1).

8. Cylindricalimplantsaccording to claim 1 to 6, **characterised in that**the wire structure(s) (2a, 2b) only run(s) at the upper end of theimplant (1).

9. Cylindrical implants according to claim1 to 8, **characterised in that** they can be captured under X-ray control without direct sight.

## Revendications

1. Implants facilement démontables essentiellement cylindriques qui sont entourés une ou plusieurs fois d'une ou plusieurs hauteurs d'une ou plusieurs structures élastiques fines sous forme de fil (2a, 2b) qui présente(nt) à une extrémité, un dispositif de capture (3a, 3b), dans lesquels une extrémité (4, 4b) ou plusieurs extrémités (4a, 4b) de la structure en forme de fil (2a, 2b) ou des structures en forme de fil (2a, 2b) est/sont fixée(s) sur ou dans l'implant, les boucles évoluant librement autour de l'implant, au moins une boucle de la ou des structure(s) en forme de fil (2a, 2b) étant prévue(s) pour réduire au moins partiellement le diamètre de l'implant lors d'une traction sur les dispositifs de capture (3a, 3b) et la ou les structure(s) sous forme de fil (2a, 2b) évoluant entre l'implant et un autre revêtement.

2. Implants cylindriques selon la revendication 1, **caractérisés en ce que** l'implant (1) est constitué d'une grille ou d'un filet en métal ou en matière plastique, doté d'une rainure ouverte.

3. Implants cylindriques selon la revendication 1 ou 2, **caractérisés en ce que** la ou les structure(s) sous forme de fil sont en métal ou en plastique.

4. Implants cylindriques selon les revendications 1 à 3, **caractérisés en ce que** le dispositif de capture (3a, 3b) est constitué comme une extrémité agrandie, un oeillet ou une boucle.

5. Implant cylindrique selon les revendications 1 à 4, **caractérisé en ce que** la ou les structure(s) en forme de fil (2a, 2b) entoure(nt) l'implant (1) uniquement sur une hauteur.

6. Implants cylindriques selon les revendications 1 à 5, **caractérisés en ce que** la ou les structure(s) (2a, 2b) entoure(nt) l'implant en formant une spirale.

7. Implants cylindriques selon les revendications 1 à 6, **caractérisés en ce que** la ou les structure(s) en forme de fil (2a, 2b) sont aménagées sur la longueur totale de l'implant (1).

8. Implants cylindriques selon les revendications 1 à 6, **caractérisés en ce que** la ou les structure(s) en forme de fil (2a, 2b) évolue(nt) uniquement sur l'extrémité supérieure de l'implant (1).

9. Implants cylindriques selon les revendications 1 à 8, **caractérisés en ce qu'**ils peuvent être détectés par contrôle radiographique sans visualisation directe.
